# EUROPEAN PATENT APPLICATION

(11) **EP 2 514 832 A1**
(43) Date of publication of application: **24.10.2012**
(21) Application number: 11462003.2
(22) Date of filing: 29.03.2011
(51) Int. Cl.: C12Q 1/44

(54) **Method for colorimetric determination of cPNMP specific enzyme activity via coupled enzymatic reactions**

(71) Applicant: Semmelweis Egyetem, 1085 Budapest (HU)
(72) Inventor: Lacza, Zsombor, 1121 Budapest (HU); Hornyák, István, 3530 Miskolc (HU)
(74) Representative: Török, Ferenc

(57) **Abstract**

The invention relates to a method for determination of phosphodiesterase enzyme (PDE) activity or the inhibitory activity of an inhibitor candidate molecule by colorimetric assay. The invention primarily relates to the coupled usage of enzymes and enzyme specific substrates for the colorimetric determination of the enzyme activity in the enzyme catalyzed conversion of cyclic purine nucleoside monophosphate (cPNMP) to opened PNMP (e.g. cGMP to GMP). The invention is based on the discovery that four or five enzymes can be coupled in a one pot reaction to provide a final colored mixture that is suitable for qualitative or quantitative analysis.

## Description

### Field of the invention

The invention primarily relates to the coupled usage of enzymes and enzyme specific substrates for the colorimetric determination of the enzyme activity in the enzyme catalyzed conversion of cyclic purine nucleoside monophosphate (cPNMP) to opened PNMP (e.g. cGMP to GMP). The invention is based on the discovery that four or five enzymes can be coupled in a one pot reaction to provide a final colored mixture that is suitable for qualitative or quantitative analysis.

### State of the art

The quantitative colorimetric analysis of enzyme activity has numerous widespread applications, usually involving two or more enzymes with an enzyme or substrate specific color reaction at the end. These reactions are usually put together in a kit to ensure that the appropriate components are used. The colorimetric determination is usually the most convenient method for the rapid determination of enzyme or inhibitor activity.

The emerging importance of phosphodiesterase enzyme (PDE) inhibitors and the limited availability of colorimetric determination assay kits cause difficulties in the rapid determination (high throughput screening) of the inhibitor activity of any possible inhibitor species, and so also to the production of new generation active PDE inhibitor compounds. The PDE-5 (phosphodiesterase type 5) inhibitors are especially in the centre of interest since they inhibit the degradation of cGMP which plays an important role in sexual function.

The current assay tests on the market provide determination via scintillation or colorimetric or luminometric method using one of the side products of the enzymatic coupled reactions. The colorimetric method is applied in the BIOMOL GREEN™ Quantizyme Assay System kit (ENZO Life Sciences) for the determination of the enzyme activity in the conversion of cyclic purine nucleoside monophosphate (cPNMP) to purine nucleoside monophosphate (PNMP). The PDE activity determination in this particular assay is based on the quantitative analysis of the phosphate side product of the conversion of purine nucleoside monophosphate (PNMP) to purine nucleoside (PN), so in this method a two step one stage enzymatic reaction is used. Another PDE assay kit is the PDE-Glo™ Phosphodiesterase Assay (Promega) in which the enzyme is not supplied, and a luminometer is required. This assay kit is rather a quality check for the PDE enzyme extracted by the user. The disadvantage of these kits is that they can be applied only for water soluble inhibitors or with maximum 10% organic solvent (e.g. EtOH, MeOH) or with low emulgent content (0.01-0.2%) but most of the usual inhibitors do not solve in water, so higher amounts of emulgents and/or organic solvents should be used, which can shift the reaction to an unwanted equilibria and usually contain traces of phosphate that reduces the accuracy (the phosphate traces only cause problems using the ENZO Life Sciences's kit). The main problem with the scintillation method is the need of specific and also expensive radioactive isotopes and the specific equipment for the use. In a particular assay kit the enzyme is not supplied, and the usability of emulgents (Tween 20™ recommended maximum 0.1%) and organic solvents (ethanol recommended maximum 5%) and buffers (Tris recommended maximum 50 mM) are also limited. (Amersham Phosphodiesterase [³H]cAMP SPA enzyme assay Phosphodiesterase [³H]cGMP SPA enzyme assay from GE Healthcare) In the present invention the advances of the mentioned assays (supplied enzyme, availability for high throughput screening, easy colorimetric evaluation and versatile function) are used in a one pot reaction.

The degradation starting from GMP can be seen on the following diagram [J. Physiol. 2004; 555(Pt 3):589-606].

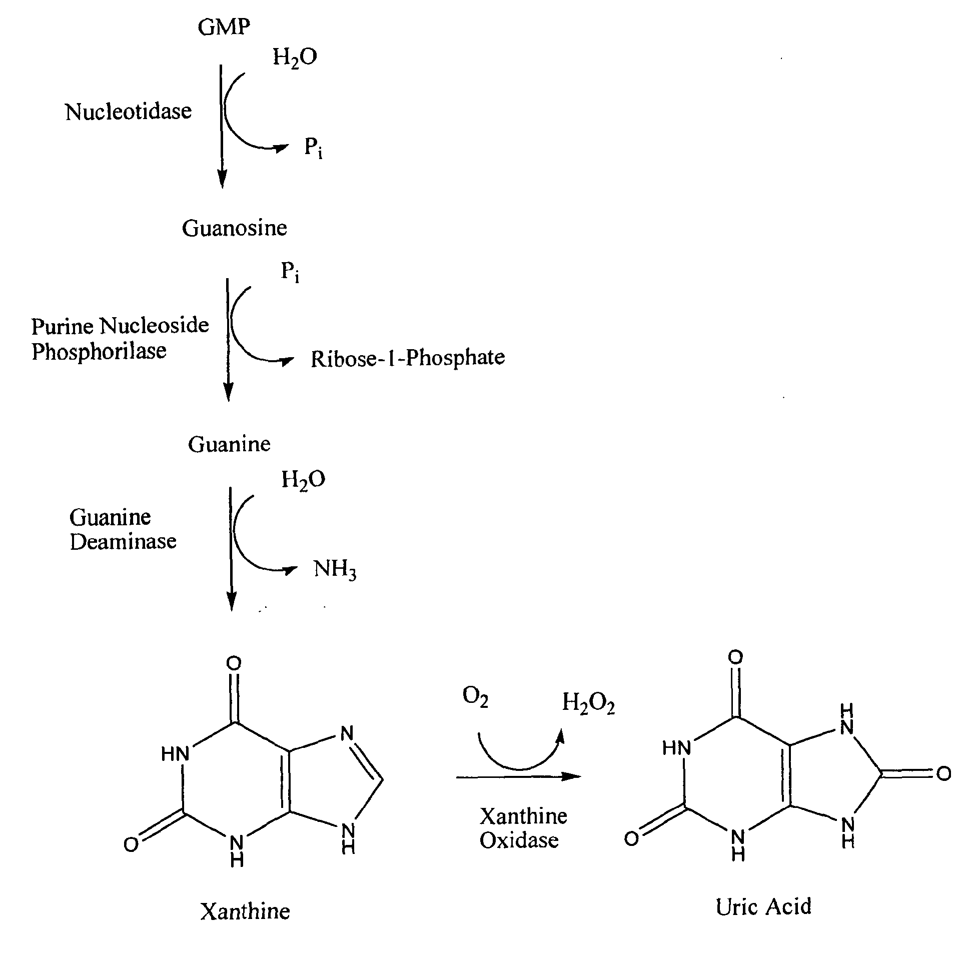

The above pathway is known only from living organisms and it has not been applied yet in *in vitro* enzyme activity determinations.

### Summary of the invention

1. The invention relates to a method for determination of phosphodiesterase enzyme (PDE) activity in a colorimetric assay, said method comprising the steps of
   a) providing a reaction mixture comprising the following substances:
      - a cyclic purine nucleoside monophosphate substrate or a salt thereof,
      - a PDE enzyme,
      - a nucleotidase enzyme,
      - a purine nucleoside phosphorylase enzyme,
      - and one of the following reagent combinations:
         i) a purine deaminase enzyme deaminating an amino-containing purine derivative and a colorimetric reagent by which the formed NH₃/NH₄⁺ undergoes a reaction resulting in a derivative having a detectable color,
         ii) a purine deaminase enzyme deaminating an amino-containing purine derivative and a purine oxidizing enzyme and a colorimetric reagent which forms during the enzymatic oxidation a derivative having a detectable color,
         iii) a purine oxidizing enzyme and a colorimetric reagent which forms during the enzymatic oxidation a derivative having a detectable color;
   b) allowing said enzymes to react (preferably in a mixing equipment, e.g. in a termostated plate shaker, preferably between 30 and 40 °C, e.g. at 37°C; for 0.25 to 3 hours, e.g. for about 60 minutes in case of one stage reaction and preferably for about two times 30 minutes in case of two stage reaction);
      c1) in case of reagent combination i) a change in the detectable color is assessed; or
      c2) in case of reagent combinations ii) or iii) calculating the activity of the PDE on the basis of the absorbance value measured at a wave length of the said detectable color.
2. Another embodiment is a method for determination of the inhibitory activity of an inhibitor candidate molecule by colorimetric assay, said method comprising the steps of
   a) providing a reaction mixture comprising the following substances:
      - inhibitor candidate molecule,
      - a cyclic purine nucleoside monophosphate substrate or a salt thereof,
      - a phosphodiesterase enzyme (PDE) enzyme,
      - a nucleotidase enzyme,
      - a purine nucleoside phosphorylase enzyme,
      - and one of the following reagent combinations:
         i) a purine deaminase enzyme deaminating an amino-containing purine derivative and a reagent by which the formed NH₃/NH₄⁺ undergoes a reaction resulting in a derivative having a detectable color,
         ii) a purine deaminase enzyme deaminating an amino-containing purine derivative and a purine oxidizing enzyme and a colorimetric reagent which turns during the oxidation into a derivative having a detectable color,
         iii) a purine oxidizing enzyme and a colorimetric reagent which turns during the oxidation into a derivative having a detectable color;
   b) allowing said enzymes to react (preferably in a mixing equipment, e.g. in a termostated plate shaker, preferably between 30 and 40 °C, e.g. at 37°C; for 0.25 to 3 hours, e.g. for about 60 minutes in case of one stage reaction and preferably for about two times 30 minutes in case of two stage reaction);
      c1) in case of reagent combination i) a change in the detectable color is assessed; or
      c2) in case of reagent combinations ii) or iii) calculating the inhibitory activity of the inhibitor candidate molecule on the basis of the absorption value measured at the wave length of the said detectable color.

In case of the above methods, for qualitative determination of the activity, reagent combination i) is applied in the case of a cyclic purine nucleoside monophosphate substrate which has an amino group in the purine part of the molecule.

In case of the above methods, for quantitative determination of the activity, reagent combination ii) is applied in the case of a cyclic purine nucleoside monophosphate substrate which has an amino group in the purine part of the molecule.

In case of the above methods, for the quantitative determination of the activity, reagent combination iii) is applied in the case of a cyclic purine nucleoside monophosphate substrate which does not have amino group in the purine part of the molecule.

In case of above method 1, part ii) and iii), during the calculation of the activity of the PDE the measured absorbance is compared to that absorbance which was measured by the use of the same substrate and enzymes as given in method 1 but the applied PDE is a control PDE with known activity.

In case of above method 2, part ii) and iii), during the calculation of the inhibitory activity the measured absorbance is compared to that absorbance which was measured by the use of the same substrate and enzymes as given in method 2 but in lack of inhibitor candidate molecule.

In preferred embodiments
- the cyclic purine nucleoside monophosphate substrate or a salt thereof is cGMP, cAMP, cIMP or cXMP or a salt thereof, preferably cGMP or a salt thereof;
- the PDE enzyme is PDE-5 enzyme;
- the nucleotidase enzyme is 5'-nucleotidase enzyme;
- the a purine deaminase enzyme is guanine deaminase enzyme;
- the purine oxidizing enzyme is xanthine oxidase enzyme.

In a preferred embodiment of the qualitative determination the colorimetric reagent is Nessler's reagent.

In a preferred embodiment of the quantitative determination the colorimetric reagent is a tetrazolium salt, e.g. MTT, or a chlorobenzenone-indophenol derivative, e.g. DCIP.

According to our invention the combination of the appropriate enzymes and substrates make it possible to ensure that the results are visible and also easy to evaluate. The invented system can be applied for both water soluble and insoluble inhibitors because with the usage of these substrates and enzymes we can both use water and organic solvents (e.g. EtOH up to 30% and emulgents up to 10%)

### Detailed description of the invention

The following enzyme reactions are applied in the methods according to the invention:
1. A substrate (a cyclic purine nucleoside monophosphate, cPNMP or a salt thereof, preferably sodium salt) is mixed with a PDE enzyme that opens the phosphate cycle, so the corresponding purine nucleoside monophosphate (PNMP) is formed.
2. A nucleotidase enzyme cleaves the phosphate from the opened nucleoside monophosphate and the corresponding nucleoside is formed (e. g. guanosine, inosine, adenosine, xanthosine).
3. A purine nucleoside phosphorylase (PNP) enzyme produces the corresponding purine derivative (e.g. guanine, hypoxanthine, adenine, xanthine) in the third step.
4. By a purine deaminase (PDA) (which can deaminate an amino-containing purine derivative) a deaminated purine derivative is formed (e.g. xanthine, hypoxanthine, allopurinol).The NH₃ releasing in this step can be the basis of a qualitative analysis.
5. In the fifth step a purine oxidizing enzyme [which enzymatically oxidizes both the amino containing and the deaminated purine derivative, (but with different selectivity) like the derivative formed in the above step 4], e.g. xanthine oxidase (other name: xanthine oxidoreductase, XOR) enzyme oxidizes the corresponding purine derivative coupled with the production of a side product having a specific and detectable color, e.g. by the reduction of a tetrazolium salt into the relating formazan compound [e.g. the yellow 3-(4,5-dimethythiazol-2-yl)-2,5-diphenyl tetrazolium bromide (MTT) is reduced into the corresponding purple formazan in a redox reaction]. The intensity of the color is proportional with the amount of the opened cPNMP in the first step, i.e. with the activity of the PDE applied.

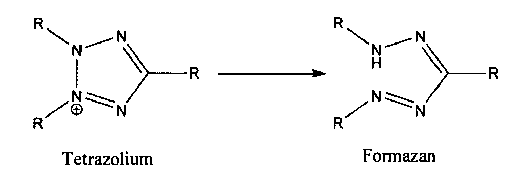

The above 1 to 4 steps (in case of qualitative assay) or 1 to 5 steps (in case of quantitative assay) can practically be combined in one or more stages. Preferably, in case of quantitative assay, steps 1 and 2 are combined into one stage (where the first and second enzymes are added together) and the final steps of 3, 4 and 5 are combined into a second stage (where the third, fourth and fifth enzymes are added together).

For qualitative assay it is also advantageous to use the above two stage one pot method (without step 5, of course). The reaction also works as a one stage one pot reaction (where all the four enzymes are added together) but with reduced intensity.

In a preferred embodiment the cGMP-GMP-guanosine-guanine-xanthine-uric acid enzymatic decomposition is applied for the analysis. This preferred embodiment of the invention is detailed in the following reaction scheme:
1. In the first step a PDE enzyme catalyzes the cGMP to GMP conversion: 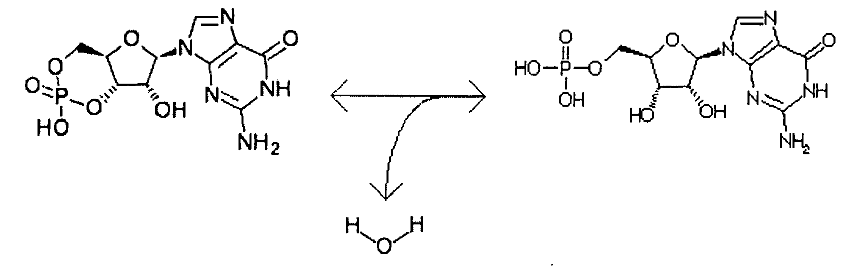
2. In the second step a nucleotidase catalyzes the GMP to guanosine conversion: 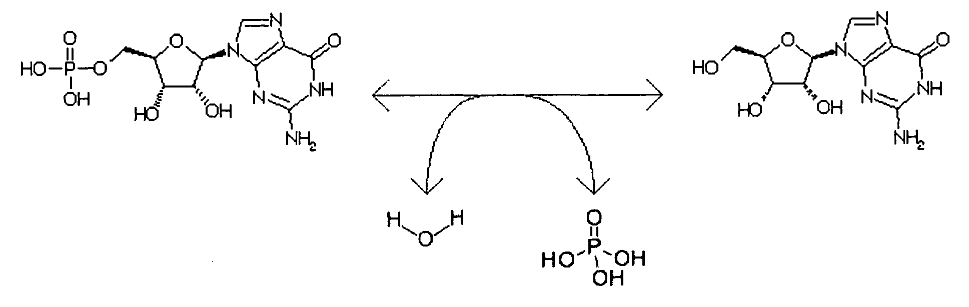 In a preferred embodiment the above two reactions are involved in a first stage, where the ingredients are incubated preferably for 10 to 180 minutes at 30 to 45 °C, more preferably for 30 to 90 minutes at about 37°C.
3. In the third step purine nucleoside phosphorylase enzyme catalyzes the guanosine to guanine conversion. 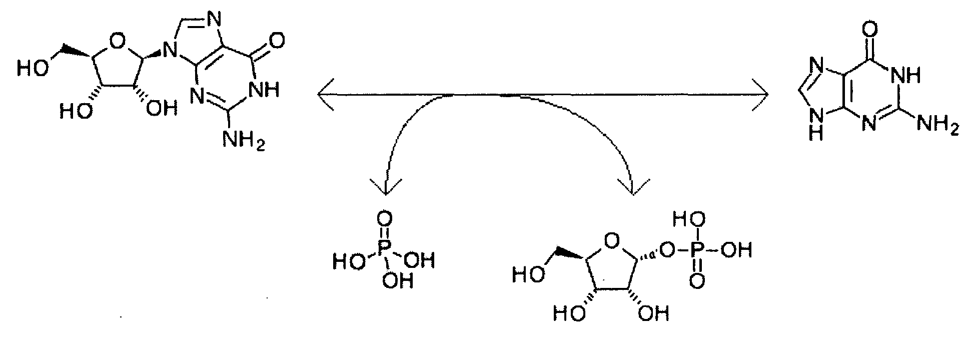
4. In the fourth step guanine deaminase (GDA) enzyme catalyzes the guanine to xanthine conversion.

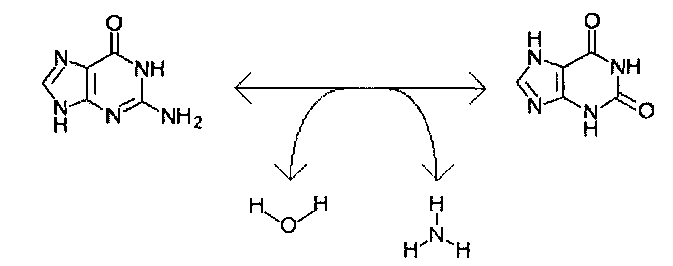

In this reaction NH₃ is a side product that forms NH₄⁺ in the aqueous solution that can give brown insoluble pellets with Nessler's reagent. This reaction (where a complex is formed with the produced NH₃/NH₄⁺ is suitable only for qualitative analysis (a slurry precipitation is formed in which no precise concentration-absorption function can be set up).

In the case of the above-mentioned preferred embodiment the above two reactions are regarded as a second stage, where the ingredients are incubated preferably for 10 to 300 minutes at 30 to 45°C, more preferably for about 30 to 90 minutes at about 37°C.

However, the above four reactions can also be put together in a "one pot one stage" reaction with all the four enzymes in a consecutive four step reaction (where all the four enzymes are added together) but this embodiment results in a reduced intensity.

If a quantitative analysis is aimed, then a further enzyme should be applied, namely:
5. In a fifth step xanthine oxidase (XOR) enzyme is applied, which catalyzes the xanthine to uric acid transformation.

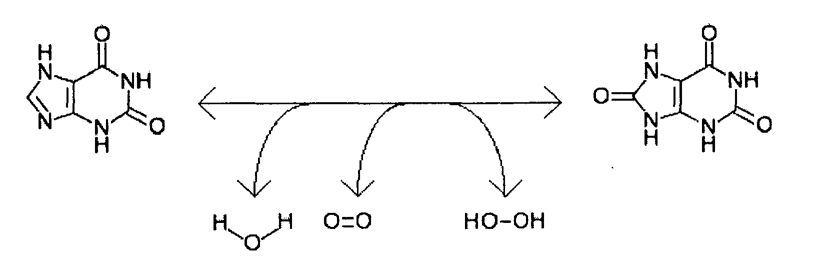

When this reaction takes place the used xanthine oxidase enzyme produces the purple formazan in a coupled reaction with a tetrazolium salt. The formazan production is inversely proportional to the inhibition if an inhibitory compound is applied in the first step for the inhibition of the PDE activity. As it was discussed above, the oxidation by XOR is coupled with the reduction of the yellow 3-(4,5-dimethythiazol-2-yl)-2,5-diphenyl tetrazolium bromide (MTT) into the corresponding purple formazan. The last reaction can be modified according to one of the suitable H₂O₂ determinations, e.g. the color reaction with horseradish peroxidase and TMB (N,N,N',N'-Tetramethylbenzidine). However, the interference due to the different substrates needs to be considered in order to choose the best method.

The above 1 to 5 steps can practically be combined in one or more stages in case of quantitative assay. Preferably, steps 1 and 2 are combined into one stage (where the first and second enzymes are added together) and the final steps of 3, 4 and 5 are combined into a second stage (where the third, fourth and fifth enzymes are added together).

The above one pot, two stage method, with the application of all the five enzymes provides a precise concentration-absorption function which can be used for different type quantitative assays as follows:
a) It can be used for studying the inhibitor activity of an inhibitor candidate compound (candidate compound in the following parts). In this case the PDE, the inhibitor candidate and the substrate are combined with each other and the above consecutive enzymatic reactions are carried out. The inhibitory activity can be calculated on the basis of the detected color intensity (absorbance value). In such an arrangement the zero absorbance means a 100% inhibitory effect and the 100% absorbance (compared to the control absorbance) means 0 % inhibitory effect. The control absorbance means the absorbance of such a combination where the same amount of substrate and PDE are applied without any inhibitor compound (this measured control absorbance is regarded as 100%).
b) On the basis of studying the developing of the color (taking the time-absorbance kinetic curve) the activity of the applied PDE also can be determined.

Beyond the number and sequence of the steps, the added enzymes and substrates, some other additives (reaction promoters, buffer solutions, one or more emulgents or organic solvents) can be applied to enhance the color intensity and to eliminate the formation of undesired side products. This type of optimization is within the knowledge of a skilled person. For example the following additives can be applied to achieve the desired results:
a) Reaction promoters (e.g. phosphate anion, glutathione, magnesium cation, activating compounds) can be applied for enhancing the reaction speed.
b) Buffer solutions (e.g. Tris, Tris.HCl, HEPES, TAPS, PBS) can be applied to ensure the desired pH value/range.
c) Emulgents (e.g. Tween™ emulgents, PEG, Triton-X) can be applied to make the phase-transfer more effective.
d) Organic solvents (e.g. EtOH, DMSO, MeOH, iPrOH, glycerol) can be applied for promoting the solubilization of non-water soluble ingredients.

The determination of the applied activities/units is within the knowledge of a skilled person. However, as a preferred embodiment, the following activities/ units and other reaction conditions can be applied in case of the cGMP-GMP-guanosine-guanine-xanthine-uric acid system.
a) The concentration of the cGMP as a starting material between 100 pM and 50 mM, preferably between 100 µM and 10 mM in an aqueous solution.
b) In a one pot one stage reaction (qualitative assay) the following enzyme activities/ units can be applied (unit is abbreviated as U):
   i) PDE of 1 U to 1000 U, preferably 20 U to 500 U. One unit is defined as the amount of enzyme that will hydrolyze 1.0 pmol cPNMP per min at 25°C, pH 7.2.
   ii) Nucleotidase enzyme (that converts GMP to guanosine) of 500 U to 1000000 U, preferably 5000 U to 100000 U. One unit is defined as the amount of enzyme that will release 1.0 pmol purine nucleoside per min at 30°C, pH 7.4.
   iii) Purine nucleoside phosphorylase (PNP) of 0.0001 to 1 U, more preferably 0.001 to 0.1 U. One unit is defined as the amount of enzyme that converts 1 µmol inosine to hypoxanthine per min at 25°C pH 7.6.
   iv) Guanine deaminase (GDA) of 0.0001 to 1 U, more preferably 0.001-0.1 U. One unit is defined as the amount of enzyme that deaminates 1.0 µmole of guanine to xanthine per min at pH 8.0 at 25°C.
c) In case of a preferred embodiment for the qualitative assay with four enzymes (one pot two stage reaction), in the first stage:
   i) PDE of 1 U to 1000 U, preferably 20 U to 500 U. One unit is defined as the amount of enzyme that will hydrolyze 1.0 pmol cPNMP per min at 25°C, pH 7.2.
   ii) Nucleotidase enzyme (that converts GMP to guanosine) of 500 U to 1000000 U, preferably 5000 U to 100000 U. One unit is defined as the amount of enzyme that will release 1.0 pmol purine nucleoside per min at 30°C, pH 7.4.

In this first stage the two enzymes are incubated with the substrate at 25 to 45 °C preferably about 37°C for a time interval of 10 to 180 minutes, preferably 30 to 90 minutes in simple aqueous solution or in the presence of a buffer solution and one or more emulgents or organic solvents. The used emulgent is preferably Tween 20™ of 0.1 to 10% by volume, the used buffer is Tris-HCl or PBS, the pH value is between 6 and 8.

In the second stage of this embodiment the following enzymes are applied:
iii) Purine nucleoside phosphorylase (PNP) of 0.0001 1 to 1 U, preferably 0.001 to 0.1 U. One unit is defined as the amount of enzyme that converts 1 µmol inosine to hypoxanthine per min at 25°C pH 7.6.
iv) Guanine deaminase (GDA) of 0.0001 to 1 U, more preferably 0.001 to 0.1 U. One unit is defined as the amount of enzyme that deaminates 1.0 µmole of guanine to xanthine per min at pH 8.0 at 25°C.

The time interval for the incubation in the second stage for the quantitative analysis is 10 to 300 minutes, preferably 30 to 90 minutes.
d) In a one pot one stage reaction (quantitative assay) the following enzyme activities/concentrations can be applied:
   i) PDE of 1 U to 1000 U, preferably 20 U to 500 U. One unit is defined as the amount of enzyme that will hydrolyze 1.0 pmol cPNMP per min at 25°C, pH 7.2.
   ii) Nucleotidase enzyme (that converts GMP to guanosine) of 500 U to 1000000 U, preferably 5000 U to 100000 U. (One unit is defined as the amount of enzyme that will release 1.0 pmol purine nucleoside per min at 30°C, pH 7.4).
   iii) Purine nucleoside phosphorylase (PNP) of 0.0001 to 1 U, preferably 0.001- to 0.1 U. One unit is defined as the amount of enzyme that converts 1 µmol inosine to hypoxanthine per min at 25°C pH 7.6.
   iv) Guanine deaminase (GDA) of 0.0001 to 1 U, preferably 0.001 to 0.1 U. One unit is defined as the amount of enzyme that deaminates 1.0 µmole of guanine to xanthine per min at pH 8.0 at 25°C.
   v) Xanthine oxidase (XOR) of 0.0001 to 1 U, preferably 0.001 to 0.1 U. One unit is defined as the amount of enzyme that converts 1 µmol xanthine to uric acid per min at 25°C pH 7.5
   e) In a preferred embodiment of the quantitative assay the enzymes of d)i) and d)ii) are applied out together in a first stage, and enzymes of d)iii), d)iv) and d)v) are applied out together in a second stage. The applied conditions and other reagents are the same as in point
   f) The used colorimetric reagent to develop the detected color in the above qualitative assays is an NH₃ specific reagent, preferably Nessler's reagent. The concentration of the used colorimetric reagent is between 0.1 and 10 % by volume, preferably between 0.5 and 5 by volume. The time for incubation is between 10 and 300 minutes, preferably between 30 and 90 minutes.
   g) The used colorimetric reagent to develop the detected color in the above quantative assays is a tetrazolium compound [preferably MTT (3-(4,5-dimethythiazo)-2-yl)-2,5-diphenyl tetrazolium bromide or TTC (triphenyl tetrazolium chloride) ] or a chlorobenzenone-indophenol derivative [e.g. DCIP (2,6-dichlorobenzenone-indophenol sodium salt]. The applied concentration is of 0.1 mg/ml to 20 mg/ml, preferably 1 mg/ml to 10 mg/ml.

### Definitions

### Substrate

In the starting point of the above enzymatic reactions a cyclic purine nucleoside monophosphate (cPNMP) is applied as substrate. This cyclic compound is opened by the applied PDE enzyme. The substrate can be e.g. cGMP, cAMP, cIMP, cXMP.

### 1. cGMP

cGMP (cyclic guanosine monophosphate) is a cyclic nucleoside derived from a phosphate a guanine and a ribose unit. It is also an important second messenger in biological systems and is synthesized from guanosine triphosphate (GTP) by the guanylate cyclase (GC) enzyme family. This family consists of the particulate transmembrane (pGC) class, which is induced by peptide ligands, and the soluble form (sGC), which is induced by nitric oxide and resides in the cytosol. A range of biological functions are triggered by cGMP, such as gating of ion channels or activating protein kinases. The activity of cGMP is regulated by its conversion to guanosine monophosphate (GMP) by the enzyme class of the phosphodiesterases (PDE). The biochemical pathway of cGMP provides potential drug targets for a range of diseases. For example, currently, PDE-inhibitors such as sildenafil are utilized in clinical practice to treat erectile dysfunction and pulmonary hypertension. (Journal of Chromatography B Volume 878, Issues 3-4, 1 February 2010, Pages 487-491). It is instable both in vivo and in vitro, the most widespread in vitro form is the sodium salt of cGMP. In vivo it is a second messenger, and is synthesized by guanylate cyclase, and is degraded according to the purine metabolism pathway. The enzyme responsible for the cGMP cleavage is PDE, especially PDE type 1,2,3,5,6,9,10.

### 2. cAMP

cAMP (cyclic adenosine monophosphate) is a cyclic nucleoside derived from a phosphate an adenine and a ribose unit. It is instable both in vivo and in vitro. In vivo it is a second messenger, and is synthesized by adenylate cyclase, and is degraded according to the purine metabolism pathway.

### 3.cIMP

cIMP (cyclic inosine monophosphate) is a cyclic nucleoside derived from a phosphate a hypoxanthine and a ribose unit. It is also degraded according to the purine metabolism pathway.

### 4.cXMP

cXMP (cyclic xanthosine monophosphate) is a cyclic nucleoside derived from a phosphate a xanthine and a ribose unit. It is also degraded according to the purine metabolism pathway.

### Phosphodiesterase enzymes (PDE)

At least 11 different gene families of PDEs in mammals were identified and characterized. These enzymes cleave the phosphodiester bond hydrolyzing the 3',5'-cyclic nucleotide to its corresponding 5'-monophosphate. PDE families 1, 2, 3 and 10 hydrolyze both cGMP and cAMP; PDE families 4, 7 and 8 prefentially cleave cAMP and PDE families 5, 6 and 9 specifically hydrolyze cGMP. The activity of PDEs is crucial for regulation of the intracellular concentration of cyclic nucleotides. (Journal of Physiology and Pharmacology 2005, 56, Supp 2, 158-34)

### PDE-5

PDE-5 (3',5'-cyclic-GMP phosphodiesterase EC 3.1.4.35) is the predominant phosphodiesterase in the corpus cavernosum. However, at least 11 families of PDE have been identified in mammals. Some types of PDE are specific for either cyclic adenosine monophosphate (cAMP) or cGMP, and some degrade both. PDE11, for example, degrades both cAMP and cGMP, whereas PDE4 is specific for cAMP, and PDE-5 is specific for cGMP. (International Journal of Impotence Research (2004) 16, S4-S7)

### Nucleotidase

Generally nucleotidases are a family of enzymes, which catalyzes nucleotide hydrolysis. The reaction products are a nucleoside and a phosphate. The nucleotidases can be divided into two subcategories, the 3'-nucleotidases, and the 5'-nucleotidases. The main difference is the reaction pathway between the two families. 5' nucleotidases cleave off the phosphate from the 5' end of the sugar moiety, the 3' nucleotidases cleave off the phosphate from the 3' end of the sugar moiety.

### 5'-nucleotidase

5'-nucleotidase (EC 3.1.3.5) activity was first described in heart and skeletal muscle about 60 years ago, it catalyzes the hydrolysis of phosphate esterified at carbon 5' of the ribose and deoxyribose portions of nucleotide molecules. (Biochem J. 1992 Jul 15;285 ( Pt 2):345-65.)

### Purine Nucleoside Phosphorylase (PNP)

PNP (EC 3.2.2.1) Plasmodium falciparum purine nucleoside phosphorylase (PfPNP) has a central role in purine salvage and inhibitors of the enzyme have been shown to have antiplasmodial activity. The enzyme preferentially uses inosine as substrate, but can also use uridine, adenosine, guanosine, xanthosine. The main difference is the efficiently with the different substrates (European Journal of Medicinal Chemistry Volume 45, Issue 11, November 2010, Pages 5140-5149). The PNP enzyme catalyzes the reversible phosphorolysis of the N-ribosidic bond of 6-oxopurine deoxynucleosides and nucleosides yielding their correspondent base and ribose-1-phosphate. Crystal structures of several PNPs have been described which can be assigned to two main categories: the low-molecular-mass homotrimeric PNPs which bind to 6-oxopurines, and high-molecular-mass homohexameric PNPs that accept either 6-oxo or 6-aminopurine (such as adenosine) as substrates (J Synchrotron Radiat. 2011 January 1; 18(Pt 1): 62-65.)

### Purine deaminase enzymes

A purine deaminase enzyme (PDA) has purine deaminating activity, i.e. it deaminates an amino-containing purine derivative. It can be e.g. guanine deaminase, adenine deaminase, adenosine deaminase.

### Guanine deaminase

Guanine deaminase (GDA) or guanase (EC 3.5.4.3) is an enzyme that catalyzes the hydrolytic deamination of guanine to xanthine. This enzyme has been found in human liver, brain, and kidney. (Bioorganic & Medicinal Chemistry Letters Volume 16, Issue 21, 1 November 2006, Pages 5551-5554)

### Purine oxidizing enzymes

A purine oxidizing enzyme enzymatically oxidizes a deaminated purine derivative. It can be e.g. xanthine oxidase, aldehyde oxidase, retinal oxidase, xanthine dehydrogenase.

Xanthine oxidase or xanthine oxidoreductase (XOR) (EC 1.17.3.2 or 1.1.3.22) is part of a group of enzymes known as the molybdenum iron-sulfur flavin hydroxylases. It was first discovered in milk by Schardinger in 1902. XOR is widely distributed throughout various organs including the liver, gut, lung, kidney, heart, brain and plasma with the highest levels being found in the gut and the liver. In the myocardium, it is localized to the capillary endothelial cells. The gene encoding for XOR is located at the short arm of chromosome 2. It exists in two inter-convertible forms known as XO (EC 1.1.3.22) and xanthine dehydrogenase (XDH) (EC 1.17.1.4). Mammalian XOR is present in vivo as the dehydrogenase form but is easily converted to XO by oxidation of the sulfhydryl residues or by proteolysis. Physiologically, XOR is involved in the hydroxylation of purines, pterins, and aldehydes but its primary role is as the rate-limiting enzyme in the conversion of hypoxanthine to xanthine and xanthine to uric acid. (Vasc. Health Risk Manag. 2009; 5: 265-272.)

### One pot reaction

All the needed reagents and/or enzymes and/or substrates are put together, in one reactor, where the reactor is preferably a well or a vial. In some preferred embodiments the order of the addition of the reagents can be used to optimize the analysis. This type of optimization is within the knowledge of a skilled person.

### Reaction step

One specific reagent and/or enzyme and/or substrate mixture is allowed to form an intermediate or is allowed to achieve kinetic equilibrium before adding other or more reagents and/or enzymes and/or substrates to a multiple stage reaction.

### Reaction stage

A set of one or more reaction steps. In each stage one or more reagent and/or enzyme and/or substrate needs to be added to the mixture. In some preferred embodiments the order of the addition of the reagents can be used to optimize the analysis. This type of optimization is within the knowledge of a skilled person.

### Description of the Figures

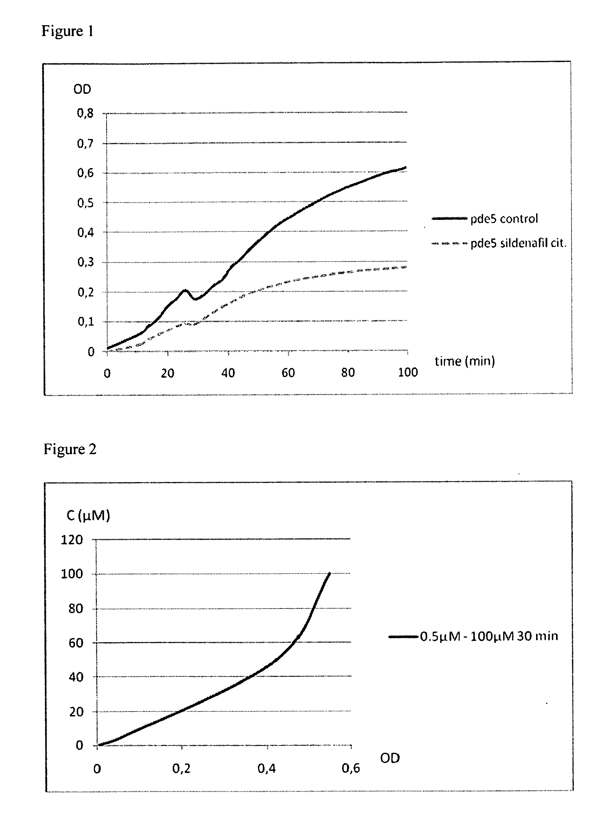

Figure 1: It shows the results of PDE-5 inhibitory measurement using cGMP as a starting material using coupled reactions (one pot two stage, quantitative reaction) in the presence and in lack of sildenafil citrate.
Figure 2: It shows the concentration - optical density diagram. The OD values were collected at different starting xanthine concentrations (from 0.5 µM to 500 µM) after 30 minutes. The results of the interval of 0.5 µM to 100 µM are shown. The concentration - optical density diagram is linear in the 0.5 µM to 60 µM xanthine concentration interval. In this particular experiment only the last substrate of the coupled enzymatic reaction is applied. The main conclusion of this color reaction is that it can be used as a reference for the coupled reactions starting with cGMP. It is advantageous to ensure by the choice of reaction conditions that the final color reaction is in the linear OD - concentration interval, because it makes easy to calculate the inhibitory effect.

### Examples:

### Example 1

### PDE activity measurement using cGMP as a starting material using coupled reactions (one pot one stage quantitative reaction)

cGMP solution of 1 mM was prepared in buffered aqueous solution (Tris.HCl, pH=7.4). 10-10 µl of this solution was added to two wells of a 96-well plate. 10 µl 5'-nucleotidase of 5 kU/µl and 5 µl PDE solution of 4 U/ml were added to both wells. After it 2 µl solution of MTT (5 mg/ml solution made in distilled water), 60 µl above Tris.HCl buffer, 1 µl purine nucleoside phosphorilase (PNP) solution of 407 U/ml, 10 µl guanine deaminase (GDA) solution of 0.2 U/ml, 1 µl xanthine oxidase (XOR) of 4 U/ml were added to the mixture. The mixture was incubated at 37°C until the purple color developed. The intensity of the developed color was proportional to the PDE-5 enzyme activity.

### Example 2

### PDE-5 activity measurement using cGMP as a starting material using coupled reactions (one pot two stage qualitative reaction)

cGMP solution of 1 mM was prepared. 10-10 µl of this solution was added to two wells of a 96-well plate. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 60 µl from the buffer (Tris-HCl pH=7.4), 1 µl purine nucleoside phosphorilase (PNP) solution of 407 U/ml and 10 µl GDA solution of 0.2 U/ml were added to the mixture. This mixture was incubated in a shaker at 37°C for half an hour (second stage). After that 10 µl Nessler's reagent was added. Brown pellets were formed.

If an inhibitor compound is mixed into the starting aqueous cGMP solution, then the formation of brown pellets proves a minimal inhibition (or there was no inhibition at all). If no change in the color of the Nessler's reagent can be seen (it is originally yellow), then the PDE-5 enzime's activity was strongly inhibited.

### Example 3

### PDE-5 activity measurement using cGMP as a starting material using coupled reactions (one pot two stage quantitative reaction)

1 mM cGMP aqueous solution was prepared. 10-10 µl of this solution was added to two wells of a 96 well plate. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 60 µl from the buffer (Tris-HCl pH=7.4), 1µl purine nucleoside phosphorilase (PNP) solution of 407 U/ml and 10 µl GDA solution of 0.2 U/ml, 1 µl xanthine oxidase (XOR) of 4 U/ml were added to the mixture. This mixture was incubated in a shaker at 37°C until the purple color developed (second stage). The colorimetric changes can be measured at 450-700 nm. The intensity of the developed color was proportional to the PDE-5 enzyme activity.

### Example 4

### PDE-5 activity measurement using cGMP as a starting material using coupled reactions (one pot two stage quantitative reaction)

1 mM cGMP aqueous solution was prepared. 10-10µl of this solution was added to two wells of a 96 well plate. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 60 µl H₂O, 1 µl purine nucleoside phosphorilase (PNP) solution of 407 U/ml and 1 µl GDA solution of 0.2 U/ml, 1 µl xanthine oxidase (XOR) of 4 U/ml were added to the mixture. This mixture was incubated in a shaker at 37°C until the purple color developed (second stage). The colorimetric changes can be measured at 450-700 nm. The intensity of the developed color was proportional to the PDE-5 enzyme activity.

### Example 5

### PDE-5 activity measurement using cGMP as a starting material using coupled reactions (one pot two stage, quantitative reaction)

50 mM cGMP aqueous solution was prepared. 10-10µl of this solution was added to two wells of a 96 well plate. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 60 µl H₂O, 1 µl purine nucleoside phosphorilase (PNP) solution of 407 U/ml and 10 µl GDA solution of 0.2 U/ml, 1 µl xanthine oxidase (XOR) of 4 U/ml were added to the mixture. This mixture was incubated in a shaker at 37°C until the purple color developed (second stage). The colorimetric changes can be measured at 450-700 nm. The intensity of the developed color was proportional to the PDE-5 enzyme activity.

### Example 6

### PDE activity measurement using cAMP as a starting material using coupled reactions (one pot two stage, quantitative reaction)

50 mM cAMP aqueous solution was prepared 10-10 µl of this solution was added to two wells of a 96 well plate. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 60 µl H₂O 1 µl purine nucleoside phosphorilase (PNP) solution of 407 U/ml and 10 µl GDA solution of 0.2 U/ml, 1 µl xanthine oxidase (XOR) of 4 U/ml were added to the mixture. This mixture was incubated in a shaker at 37°C until the purple color developed (second stage). The colorimetric changes can be measured at 450-700 nm. The intensity of the developed color was proportional to the PDE-5 enzyme activity.

### Example 7

### PDE-5 inhibitory measurement using cGMP as a starting material using coupled reactions (one pot two stage, quantitative reaction)

50 mM cGMP aqueous solution was prepared.

Well A2A3: 40 µl of the 50 mM cGMP aqueous solution was added to both wells of a 96 well plate and 10 µl 20 % Tween 20™, 120µl H₂O were added afterwards. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 1 µl purine nucleoside phosphorilase (PNP) solution of 407 U/ml and 10 µl GDA solution of 0.2 U/ml, 1 µl xanthine oxidase (XOR) of 4 U/ml were added to the mixture.

Well B2B3: 40 µl of the 50 mM cGMP aqueous solution was added to both wells of a 96 well plate and 10 µl 20% Tween 20™, 10µl sildenafil citrate (5 mg/15ml solution made in distilled water) and 120 µl H₂O were added afterwards. 10 µl 5'-nucleotidase of 5 kU/µl and 4 µl PDE-5 solution of 50000 U/ml were added to both wells. The mixture was incubated for half an hour (first stage). After this 2 µl solution of MTT (5 mg/ml solution made in distilled water), 1 µl purine nucleoside phosphorilase (PNP) solution of 407 U/ml and 10 µl GDA solution of 0.2 U/ml, 1 µl xanthine oxidase (XOR) of 4 U/ml were added to the mixture.

Both mixtures were incubated in a shaker at 37°C until the purple color developed (second stage). The colorimetric changes can be measured at 450-700 nm. The intensity of the developed color was proportional to the PDE-5 enzyme activity. The results were evaluated on an optical density (OD) - time (minute) - see the diagram on Figure 1.

According to the results, in the present example the color intensity is half of the control using 25µM sildenafil citrate. According to our measurements the concentration - optical density diagram is linear within the 0-0.5 optical density range, which corresponds to the 5-60 µM xanthine concentration interval with the reaction parameters shown in Example 7. The OD values were collected at different starting xanthine concentrations (from 0.5 µM to 500 µM) after 30 minutes (see Figure 2).

## Claims

1. Method for determination of phosphodiesterase enzyme (PDE) activity in a colorimetric assay, said method comprising the steps of
a) providing a reaction mixture comprising the following substances:
- a cyclic purine nucleoside monophosphate substrate or a salt thereof,
- a PDE enzyme,
- a nucleotidase enzyme,
- a purine nucleoside phosphorylase enzyme,
- and one of the following reagent combinations:
i) a purine deaminase enzyme deaminating an amino-containing purine derivative and a colorimetric reagent by which the formed NH₃/NH₄⁺ undergoes a reaction resulting in a derivative having a detectable color,
ii) a purine deaminase enzyme deaminating an amino-containing purine derivative and a purine oxidizing enzyme and a colorimetric reagent which forms during the enzymatic oxidation a derivative having a detectable color,
iii) a purine oxidizing enzyme and a colorimetric reagent which forms during the enzymatic oxidation a derivative having a detectable color;
b) allowing said enzymes to react;
c1) in case of reagent combination i) a change in the detectable color is assessed; or
c2) in case of reagent combinations ii) or iii) calculating the activity of the PDE on the basis of the absorbance value measured at a wave length of the said detectable color.

2. Method for determination of the inhibitory activity of an inhibitor candidate molecule by colorimetric assay, said method comprising the steps of
a) providing a reaction mixture comprising the following substances:
- inhibitor candidate molecule,
- a cyclic purine nucleoside monophosphate substrate or a salt thereof,
- a phosphodiesterase enzyme (PDE) enzyme,
- a nucleotidase enzyme,
- a purine nucleoside phosphorylase enzyme,
- and one of the following reagent combinations:
i) a purine deaminase enzyme deaminating an amino-containing purine derivative and a reagent by which the formed NH₃/NH₄⁺ undergoes a reaction resulting in a derivative having a detectable color,
ii) a purine deaminase enzyme deaminating an amino-containing purine derivative and a purine oxidizing enzyme and a colorimetric reagent which turns during the oxidation into a derivative having a detectable color,
iii) a purine oxidizing enzyme and a colorimetric reagent which turns during the oxidation into a derivative having a detectable color;
b) allowing said enzymes to react;
c1) in case of reagent combination i) a change in the detectable color is assessed; or
c2) in case of reagent combinations ii) or iii) calculating the inhibitory activity of the inhibitor candidate molecule on the basis of the absorption value measured at the wave length of the said detectable color.

3. The method according to claim 1 or 2 for the qualitative determination of the activity where reagent combination i) is applied in the case of a cyclic purine nucleoside monophosphate substrate which has an amino group in the purine part of the molecule.

4. The method according to claim 1 or 2 for the quantitative determination of the activity where reagent combination ii) is applied in the case of a cyclic purine nucleoside monophosphate substrate which has an amino group in the purine part of the molecule.

5. The method according to claim 1 or 2 for the quantitative determination of the activity where reagent combination iii) is applied in the case of a cyclic purine nucleoside monophosphate substrate which does not have amino group in the purine part of the molecule.

6. The method according to any of claim 1, part ii) and iii) and claims 4 and 5 where during the calculation of the activity of the PDE the measured absorbance is compared to that absorbance which was measured by the use of the same substrate and enzymes as given in claim 1 but the applied PDE is a control PDE with known activity.

7. The method according to any of claim 2, part ii) and iii) and claims 4 and 5 where during the calculation of the inhibitory activity the measured absorbance is compared to that absorbance which was measured by the use of the same substrate and enzymes as given in claim 2 but in lack of inhibitor candidate molecule.

8. The method according to any of claims 1 to 7 where
- the cyclic purine nucleoside monophosphate substrate or a salt thereof is cGMP, cAMP, cIMP or cXMP or a salt thereof, preferably cGMP or a salt thereof;
- the PDE enzyme is PDE-5 enzyme;
- the nucleotidase enzyme is 5'-nucleotidase enzyme;
- the a purine deaminase enzyme is guanine deaminase enzyme;
- the purine oxidizing enzyme is xanthine oxidase enzyme.

9. The method according to any of claim 1, part i), claim 2, part i) and claims 3 and 8, where the colorimetric reagent is Nessler's reagent.

10. The method according to any of claim 1, part ii) and iii), claim 2, part ii) and iii) and claims 4 to 8, where the colorimetric reagent is a tetrazolium salt, e.g. MTT, or a a chlorobenzenone-indophenol derivative, e.g. DCIP.
